# EUROPÄISCHE PATENTSCHRIFT

(11) **EP 2 741 734 B1**
(45) Veröffentlichungstag und Bekanntmachung des Hinweises auf die Patenterteilung: **09.03.2016**
(21) Anmeldenummer: 12735126.0
(22) Anmeldetag: 09.07.2012
(51) Int. Cl.: A61K 9/00, A61K 9/20, A61K 9/70

(54) **VERFAHREN ZUR HERSTELLUNG UND KONTROLLE VON ORALEN WIRKSTOFF-FILMEN**
METHOD FOR PRODUCING AND MONITORING ORAL ACTIVE INGREDIENT FILMS
PROCÉDÉ DE PRODUCTION ET DE CONTRÔLE DE FILMS ORODISPERSIBLES

(30) Priorität: 12.08.2011 DE 102011080870; 16.12.2011 DE 102011088909
(43) Veröffentlichungstag der Anmeldung: 18.06.2014
(73) Patentinhaber: tesa SE, 22848 Norderstedt (DE); Labtec GmbH, 40764 Langenfeld (DE)
(72) Erfinder: BRAUN, Sebastian, 42929 Wermelskirchen (DE); BREITENBACH, Armin, 51371 Leverkusen (DE); SCHLIEPHACKE, Ralf, 25524 Itzehoe (DE)
(86) Internationale Anmeldenummer: PCT/EP2012/063405
(87) Internationale Veröffentlichungsnummer: WO 2013/023841

(56) Entgegenhaltungen:
- EP-A1- 0 219 762
- EP-A2- 1 306 071
- WO-A2-2008/065144
- US-A1- 2005 233 000
- US-A1- 2006 222 702

## Beschreibung

Die Erfindung betrifft ein Verfahren zur Herstellung und Kontrolle von oralen Wirkstoff-Filmen.

Orale Wirkstoff-Filme (Englisch "Oral Dissolvable Films: ODFs") stellen eine vergleichsweise neue Technologie zur oralen Verabreichung von Wirkstoffen dar. Diese Filme sind hauchdünne, ein- oder mehrschichtige Polymerfilme, die den Mund sowohl zur Applikation als auch zur Wirkstoffabsorption nutzen.

Der Film wird unter Verwendung von hydrophilen Polymeren wie Cellulose oder Maltodextrin hergestellt. Der Wirkstoff kann in der Matrix aus den Polymeren gelöst, emulgiert oder dispergiert vorliegen. Er kann auch komplex gebunden sein. Des Weiteren kann der Wirkstoff auf der Matrix in einer weiteren Schicht aufgebracht sein. Wichtig ist, dass sich die Polymere schnell auf der Zunge oder in der Mundhöhle auflösen und dabei den Wirkstoff freisetzen, wenn er mit einer Flüssigkeit in Kontakt kommt.

Die Absorption von Arzneistoffen in der Mundhöhle erfolgt in erster Linie über die bukkale und sublinguale Schleimhaut. Die sehr gute Durchblutung der Mundschleimhaut wird durch ein dichtes Netzwerk von Kapillaren gewährleistet. Die Diffusion eines Wirkstoffes in dieses Netzwerk der Mikrozirkulation ermöglicht einen unmittelbaren Übergang in den Blutkreislauf.

Derartige Filme beziehungsweise die Filmstreifen haben eine begrenzte flächenförmige Ausdehnung, typischerweise mit einer Größe zwischen 1 cm² und 10 cm² und eine Filmdicke von 20 bis 500 µm.

Größere Verbreitung haben die ODFs in Form von Mundhygienestreifen gefunden.

Herkömmliche Herstellverfahren für derartige ODFs umfassen die folgenden Arbeitsschritte:
1. Dosieren und Mischen der Basisrezeptur aus Cellulose oder Maltodextrin inklusive des Wirkstoffs
2. Beschichtung der Masse auf eine Hilfsträgerfolie
3. Trocknung der auf Hilfsträgerfolie beschichteten Masse
4. Schneiden und Wickeln von Mutterrollen
5. Ablängen, Vereinzeln und Einsiegeln von ODFs in einen Siegelbeutel

Der sehr wertvolle Wirkstoff wird standardmäßig schon in dem ersten Arbeitsschritt dosiert und zugegeben, so dass in allen darauffolgenden Arbeitsschritten die anfallenden Einfahr- und Abfallmaterialien mit dem Wirkstoff behaftet sind. Unnötige Verluste von dem wertvollen Wirkstoff und sehr aufwändige Reinigungs- sowie Entsorgungsarbeiten nach der Herstellung sind die Folge. Des Weiteren ist eine aufwändige Zwischenproduktkontrolle erforderlich, um die homogene Gleichverteilung des Wirkstoffs bei jedem Arbeitsschritt überwachen zu können.

Zum Aufbringen von Wirkstoffen in der Arzneimittelindustrie ist der Einsatz von Druckverfahren bekannt, und zwar vorrangig zum Markieren und Identifizieren von Pillen, Tabletten oder Kapseln. Dies kann der US 2009/026286 A1 oder der WO 2006/047695 A1 entnommen werden. Des Weiteren werden Bruchzonen auf Kapseln oder Tabletten mithilfe eines getakteten, das heißt nicht kontinuierlichen Druckverfahrens gekennzeichnet, wie die US 2007/0014852 A1 oder die WO 2006/058247 zeigen. Üblicherweise kommen Ink-Jet-Verfahren oder Tampondruckverfahren zur Anwendung. Die hierbei verwendeten Druckfarben beinhalten zumeist Wachse und/oder Fette oder Inhaltsstoffe, die die Kratzfestigkeit auf den Oberflächen erhöhen. Dies beschreibt die WO 2005/053599 A1.

Bei diesen genannten Anwendungen ist keine hohe Präzision der Druckfarbenmenge oder keine hohe Gleichmäßigkeit in der Druckfarbenübertragung auf das Substrat gefordert, welches darüber hinaus auch nicht mit einem getakteten Verfahren technisch möglich ist.

Aus der DE 34 23 328 A1 ist als kontinuierliches Druckverfahren zur Übertragung von Klebstoffen, die Wirkstoffe enthalten können, das Tiefdruck- und das Siebdruckverfahren bekannt.

Ein weiteres Problem in der Herstellung von ODFs besteht darin, dass die zwingend erforderliche Kontrolle des Wirkstoffgehalts zumeist als zerstörerische Produktprüfung stattfindet, das heißt, der einzelne ODF wird bezüglich seiner Bestandteile analysiert und dabei der festgestellte Wirkstoffgehalt mit dem vorgegeben Wirkstoffgehalt vergleichen. Wird dabei eine Abweichung festgestellt, kann dies zum Verlust einer ganzen Charge führen.

Für den sparsamen Umgang mit Wirkstoffen ist daher erforderlich, dass die Kontrolle während des kontinuierlichen Herstellungsverfahren stattfindet und dass das Kontrollverfahren möglichst umgehend, am besten sofort ein Ergebnis liefert, um auf Abweichungen des Wirkstoffgehalts durch einen Stopp des Verfahrens unmittelbar reagieren zu können. Auf diese Weise ist gewährleistet, dass die Verluste an Wirkstoffen insgesamt deutlich reduziert werden.

Eine typische Zusammensetzung eines oralen Wirkstofffilms sieht aus wie folgt:
- Pharmazeutisch wirksamer Wirkstoff 1 bis 30 Gew.-%
- Wasserlösliches Filmpolymer wie Pharmacoat oder Methocel (Hydroxyporpyl methylcellulose) 40 bis 50 Gew.-%
- Weichmacher wie Glycerol 0 bis 20 Gew.-%
- Füllstoffe, Farbstoffe, Aromastoffe und weitere Zusätze 0 bis 40 Gew.-%

Im Übrigen sei auf den Artikel "Advances in orodisperible films for drug delivery" der Autoren Hoffmann, Breitenbach und Breitkreuz aus dem Fachjournal *Expert Opinion on Drug Delivery -* 03/18/11 verwiesen, der auch unter anderem. die typischen Inhaltsstoffe der Basisfilme auf Seite 303 beschreibt und ansonsten eine sehr gute Zusammenfassung über die Herstellung und Eigenschaften verschiedener oraler Wirkstofffilme darstellt.

Aufgabe der Erfindung ist es, ein Verfahren zur Herstellung und Kontrolle von oralen Wirkstoff-Filmen anzugeben, das durch ein präzises Herstellungsverfahren und durch ein effektives Kontrollverfahren zu möglichst geringen Verlusten an Wirkstoffen führt und das Schwankungen im Wirkstoffgehalt in den oralen Wirkstoff-Filmen möglichst unterdrückt.

Gelöst wird diese Aufgabe durch ein Verfahren, wie es im Hauptanspruch niedergelegt ist. Vorteilhafte Ausführungsformen des Verfahrens sind Gegenstand der Unteransprüche.

Demgemäß betrifft die Erfindung ein Verfahren zur Herstellung und Kontrolle von oralen Wirkstoff-Filmen mit einer Basis, auf die eine zumindest einen Wirkstoff enthaltende Lösung aufgebracht wird, umfassend die Schritte:
- Dosieren und Mischen der Basisrezeptur,
- Beschichtung der Basisrezeptur auf eine Unterlage, so dass sich eine Bahn ergibt,
- gegebenenfalls Trocknung der auf der Unterlage beschichteten Basisrezepturbahn,
- Aufdrucken einer zumindest einen Wirkstoff enthaltenden Farbstofflösung nach dem Flexodruckverfahren auf der Oberseite der Basisrezepturbahn,
- Trocknung der auf der Unterlage beschichteten Basisrezepturbahn samt aufgedruckter Wirkstofflösung,
- Durchstrahlen der auf der Unterlage beschichteten Basisrezepturbahn samt aufgedruckter Wirkstofflösung von der Ober- und/oder Unterseite mittels einer Strahlung aus einer Strahlungsquelle,
- Messung der Transmission der durchgetretenen Strahlung auf der gegenüberliegenden Seite der auf der Unterlage beschichteten Basisrezepturbahn samt aufgedruckter Wirkstofflösung

Überraschenderweise wurde gefunden, dass das eigentlich in der Verpackungsmittelindustrie eingesetzte Flexodruckverfahren zur Übertragung von Arzneiwirkstoffen auf die Basisrezepturbahn die beste Herstellungsart ist.
Das Flexodruckverfahren ist ein kontinuierliches Druckverfahren, bietet die geforderte hohe Präzision und sichert die hohe Gleichmäßigkeit im Übertrag von Arzneiwirkstoffen auf die Basisrezepturbahn ab. Der einfache Aufbau eines Flexodruckwerks ermöglicht, mit geringsten Verlusten an Druckfarbenmengen einen prozesssicheren Beschichtungsprozess zu realisieren. Die Auftragsmenge kann durch unterschiedliche Auslegung der hoch präzisen Rasterwalze bestimmt werden und ermöglicht somit auch die sichere Gleichmäßigkeit der Auftragsmenge.
Nach DIN 16514: 1982-11 ist der Flexodruck ein Verfahren des direkten Hochdrucks.
Die Druckform ist aus Gummi oder elastischem photopolymerem Kunststoff. Die Einfärbung der Druckform erfolgt mittels einer geätzten oder gravierten Rasterwalze aus Stahl mit Verchromung oder Keramik (Chromoxid), deren Näpfchen direkt mit einer Kammerrakel oder einer Tauchwalze mit Druckfarbe gefüllt werden. Die überschüssige Farbe wird von der Rasterwalze mit einem Rakel abgerakelt oder (in älteren Maschinen) mit einer Gummiwalze abgequetscht. Die Druckgeschwindigkeit beträgt, je nach Art des Bedruckstoffes und Motivs, im Verpackungsdruck 100 bis 300 m/min. Haupteinsatzgebiet ist der Verpackungsdruck.

Es können auch mehrere Flexodruckwerke hintereinander angeordnet sein, um die Auftragsmenge entsprechend erhöhen zu können.
Für diesen Fall ist es vorteilhaft, wenn nach jedem Druck eine Messung der Transmission durchgeführt wird.
In einer alternativen Variante wird die bedruckte Basisrezepturbahn (nach der Messung) aufgewickelt und erneut dem einen Flexodruckwerk zugeführt. Dieser Vorgang kann mehrmals hintereinander durchgeführt werden, bis die gewünschte Menge an Wirkstoff aufgebracht ist.
Der Druck muss nicht vollflächig erfolgen, eine partielle Übertragung der zumindest einen Wirkstoff enthaltenden Farbstofflösung ist ebenfalls möglich.

Üblicherweise liegt der Anteil an Wirkstoff(en) in der zumindest einen Wirkstoff enthaltenden Farbstofflösung zwischen 0,01 und 25 Gew.-%, insbesondere 5 bis 15 Gew.-%. Der Wert von 25 Gew.-% kann aber auch - je nach Anforderung nach oben überschritten werden, falls es zur Erzielung des gewünschten Effektes notwendig ist.
Der Anteil an Farbstoff(en) in der zumindest einen Wirkstoff enthaltenden Farbstofflösung beträgt 0,01 und 10 Gew.-%, insbesondere 1 bis 5 Gew.-%. Auch der Wert von 10 Gew.-% kann aber auch - je nach Anforderung nach oben überschritten werden, falls es zur Erzielung des gewünschten Effektes notwendig ist.

Als Farbstoffe sollten nur solche eingesetzt werden, die in Arzneimitteln auch zugelassen sind. Derartige Farbstoffe finden sich beispielsweise in Listen wieder, die von der Food and Drug Administration (FDA) unter der Bezeichnung "Summary of Color Additives for Use in United States in Foods, Drugs, Cosmetics, and Medical Devices" herausgegeben werden. Unter diesen Farbstoffen seien namentlich erwähnt
- Brillantblau FCF oder E 133
   (Dihydrogen(ethyl)[4-[4-[ethyl- (3-sulfonatobenzyl)]amino]-2'-sulfonato-benzhydryliden]-cyclohexa-2,5-dien- 1-yliden]-(3-sulfonatobenzyl)ammonium, Dinatriumsalz)
- Indigokarmin oder E 132
   (Indigotin-5,5'-disulfonsäure Dinatriumsalz)

Die Trocknung der auf der Unterlage beschichteten Basisrezepturbahn samt aufgedruckter Wirkstofflösung erfolgt vorzugsweise kontinuierlich. Geeignete kontinuierliche Trocknungsverfahren sind unter anderem Heißlufttrocknung, Infrarot-Trocknung, Hochfrequenztrocknung und/oder Kombinationen aus allen Verfahren. Das Trocknungsprofil (Temperatur und Zeit) wird dabei so eingestellt, dass ein möglichst geringer Restlösungsmittelgehalt erreicht wird. Geeignete Temperaturen liegen zwischen 40 °C und 150 °C, geeignete Trocknungszeiten bei 1 bis 30 Minuten.

Die Bestimmung des Wirkstoffgehalts wird erfindungsgemäß mittels einer Farbstoffzugabe in der wirkstoffhaltigen Druckfarbenlösung und einem physikalischen Transmissionsmessverfahrens realisiert.
Mit der Transparenz beziehungsweise dem Transmissionsgrad - bisweilen auch nur kurz als Transmission bezeichnet -, der in der Regel in % angegeben wird, ist das Verhältnis der auf der Rückseite eines mit Licht durchstrahlten Körpers ankommenden Lichtleistung zu der auf der Vorderseite eintreffenden Lichtleistung gemeint. Die Transmission wird beschnitten durch Reflektion und Absorption.
Es gilt also: Transmissionsgrad = (1 - Reflektionsgrad - Absorptionsgrad).
Der Begriff der Transmission wird erfindungsgemäß dahingehend ausgeweitet, dass unabhängig von der verwendeten Strahlungsart der Verlust an Strahlung beim Durchgang durch die Basisrezepturschicht samt Farb- und Wirkstoffauftrag als Transmission bezeichnet wird.

Die Strahlungsquelle durchstrahlt die Basisrezepturbahn samt aufgedruckter farbiger Wirkstofflösung. Bei der Durchstrahlung treten Strahlungsverluste auf, die auf der gegenüberliegenden Seite der Bahn über einen entsprechenden Detektor festgestellt werden können.
Hierfür ist zunächst eine Nullmessung zu machen, das heißt, die mit der gewünschten Konzentration an Wirk- und Farbstoff bedruckte Basisrezepturbahn (vorsorglich sollte der tatsächliche Wirkstoffgehalt mit einem zweiten, anderen Messverfahren bestätigt werden), wird durchstrahlt und der Transmissionswert festgestellt.
Im weiteren Verfahren wird nun der aktuell gemessene Transmissionswert mit dem Nullwert verglichen. Bei Abweichungen des tatsächlichen Messwerts von der gewünschten Vorgabe kann unmittelbar reagiert werden, indem zum Beispiel das Verfahren gestoppt wird, um den Fehler abzustellen. Auf diese Weise werden die Verluste wirkungsvoll verringert.
Gerade wenn sich der Transmissionswert kontinuierlich ändert, beispielsweise ständig zunimmt, was auf eine Reduktion des Wirk- und Farbstoffgehalts hinweist, aber der Wert noch innerhalb der Toleranzgrenzen liegt, kann durch eine entsprechende Reaktion, also der sofortigen Anpassung des Wirk- und Farbstoffgehalts, ein Verlust an Wirkstoffen sogar vollständig vermieden werden.

Beispielsweise kann in der wirkstoffhaltigen Druckfarbenlösung ein Farbstoff enthalten sein, der die Lichtstrahlung einer roten Lichtquelle absorbiert. Wird eine geringe Menge an Farbstoff, das heißt gleichzeitig Wirkstoff bedruckt, ist die Absorption des Lichts gering und eine größere Menge an Licht wird von einer Photozelle erfasst, als wenn eine hohe Farbstoff und somit Wirkstoffmenge bedruckt würde. Der Transmissionsgrad ist direkt abhängig von der Farbstoffmenge (damit einhergehend Wirkstoffmenge) und damit ein direktes kontaktloses Prüfverfahren zur Bestimmung des Wirkstoffgehalts, ohne dass der Prüfkörper zerstört werden muss.

Dies stellt auch eine bevorzugte Ausführungsform des Verfahrens dar, wenn also als Strahlung sichtbares Licht und als Empfangseinheit eine Photozelle verwendet werden. Vorzugsweise ist das Kontrollverfahren ein optisches Verfahren.

Gemäß einer ersten Variante des Verfahrens werden nach der Beschichtung der Basisrezeptur auf eine Unterlage und der gegebenenfalls anschließenden Trocknung aus der sich ergebenden Bahn die einzelnen Abschnitte der oralen Wirkstoff-Filme möglichst verlustfrei gestanzt, und zwar entweder direkt vor dem Druckvorgang oder direkt nach dem Druckvorgang.

Gemäß einer zweiten Variante des Verfahrens werden nach Messung der Transmission der durchgetretenen Strahlung aus der Basisrezepturbahn die einzelnen Abschnitte der oralen Wirkstoff-Filme möglichst verlustfrei gestanzt.

Vorteilhafterweise besteht die Stanzvorrichtung aus einer rotativen Schneidwalze und einer gleichläufigen Gegenwalze, wobei die Basisrezepturbahn in den Spalt zwischen der Schneidwalze und der Gegenwalze geführt wird. Vorteilhafterweise befindet sich die Basisrezepturbahn auf einer Trennfolie, die über die Gegenwalze geführt wird. In dem Spalt werden dann die einzelnen Konturen der Stanzlinge vorzugsweise ohne Stanzgitter in der Bahn durchgestanzt, möglichst ohne die Trennfolie zu verletzen (dieses Verfahren ist unter dem Namen kiss-cut-Verfahren bekannt und zeichnet sich dadurch aus, dass beim Stanzen die Trennfolie nicht oder nur unwesentlich verletzt beziehungsweise angestanzt wird).

Weiterhin bevorzugt besteht die Stanzvorrichtung aus einer Hub- oder Flachstanze, in die die Basisrezepturbahn gefördert wird. Vor der Hubstanze kann ein Pendeleinzug und/oder hinter der Hubstanze kann ein Puffer vorgesehen sein, um trotz der getackteten und damit eigentlich diskontinuierlichen Arbeitsweise der Hubstanze einen kontinuierlichen Fortgang des Verfahrens sicherzustellen.

Abschließend werden vorteilhafterweise die einzelnen Abschnitte der oralen Wirkstoff-Filme in einen Beutel luft- und wasserdicht eingesiegelt.

Gemäß einer bevorzugten Ausführungsform der Erfindung werden als Strahlung Infrarotstrahlung, sichtbares Licht, Ultraviolettstrahlung und/oder radioaktive Strahlung verwendet. Die Erfindung ist aber nicht auf die genannten Strahlungsquellen eingeschränkt, auch andere sind hierfür geeignet.

Es können - je nach Breite der Bahn - neben einem auch zwei oder mehr Strahlungsempfänger eingesetzt werden. In einer Variante des Verfahrens pendelt der der Strahlungsempfänger insbesondere in Querrichtung der Basisrezepturbahn.

Gemäß einer bevorzugten Variante wird die Basisrezepturbahn auf eine Trägerfolie als Unterlage, vorzugsweise aus Polyethylen, beschichtet, die zusammen mit der Basisrezepturbahn durch das Verfahren geführt wird. Besonders bevorzugt wird als Unterlage eine Folie aus Polyester, ganz besonders vorzugsweise aus Polyethylenterephthalat (PET).
Die Dicke der Trennfolie liegt insbesondere zwischen 60 und 150 µm.

Die Dicke der Basisrezepturbahn liegt üblicherweise zwischen 25 bis 200 µm, insbesondere zwischen 65 bis 100 µm.

Die Breite der Basisrezepturbahn ist bevorzugt zwischen 20 und 35 mm, 30 mm sind besonders vorteilhaft.

Vorzugsweise wird das Verfahren kontinuierlich in einem Arbeitsgang durchgeführt.
Es ist aber auch möglich, nach der Beschichtung der Basisrezeptur auf die Unterlage, vorzugsweise Trennfolie und der sich gegebenenfalls anschließenden Trocknung der Basisrezepturbahn das Verfahren dahingehend zu unterbrechen, dass die Basisrezepturbahn zunächst zu Rollen aufgewickelt wird, beispielsweise mit Längen von 300 m bis 500 m.
Diese Rollen können dann gelagert oder an einen Weiterverarbeiter geliefert werden. Bei Bedarf werden die Rollen abgewickelt, und das erfindungsgemäße Verfahren beginnt mit dem Aufdrucken einer zumindest einen Wirkstoff enthaltenden Farbstofflösung nach dem Flexodruckverfahren auf der Oberseite der Basisrezepturbahn.

Typische Wirkstoffe sind - ohne den Anspruch der Vollständigkeit im Rahmen der vorliegenden Erfindung zu erheben:
Antiallergica, Antiarrhythmica, Antibiotica, Antidiabetica, Antiepileptica, Antihistaminica, Antitussiva, Cardiotonica, Diuretica, Hypotensiva, Narcotica, Neuro-Muskelblocker, Sexualhormone sowie Vasopressoren. Diese können je nach therapeutisch gewünschtem Ergebnis auch in Mischungen eingesetzt werden.
Als Wirkstoffe können darüber hinaus erfindungsgemäß die aus Mundhygienestreifen bekannte Substanz Menthol eingesetzt werden, daneben auch andere Geschmacks-, Aroma- oder Duftstoffe, wie sie im Bereich der Mundhygiene verwendet werden.

Typische Polymere zur Bildung des Basisrezepturfilms sind Cellulosederivate wie Hydroxypropylmethylcellulose, Hydroxyethylcellulose und/oder Hydroxypropylcellulose sowie Maltodextrin. Die Polymere können dabei einzeln oder in beliebigen Mischungen eingesetzt werden.

Als Strahlungsquellen können Lichtquellen, die in dem gewünschten Wellenlängenbereich emittieren eingesetzt werden. Daneben kommen auch radioaktive Strahler zur Verwendung.

Als Empfangseinheit dienen Photozellen. Bei radioaktiver Strahlung genügen ein oder mehrere Geigerzähler zur Detektion der Strahlung.

Durch die Kombination des sehr präzisen und zuverlässigen Flexodruckverfahrens mit dem inline-Messverfahren, das ein sofortiges Ergebnis liefert, vermeidet die Erfindung übermäßige Verluste an Wirkstoff und die daraus resultierenden aufwändigen Reinigungs- sowie Entsorgungsarbeiten bei Fehlproduktionen sowie die Zwischenproduktkontrollen,
Zusammenfassend hat das Verfahren einen einfachen und kompakten Maschinenaufbau und ermöglicht eine schnelle Umrüstung auf andere arzneimittelhaltige Druckfarben, wobei eine Cross-Kontamination unterschiedlicher Wirkstoffe vermieden werden kann.
Je später im laufenden Verfahren der wertvolle Wirkstoff aufgebracht wird, umso geringer sind die Verluste desselben für den Fall, dass die Konzentration vom vorgegeben Wert abweicht.
Aufgrund der sehr vorteilhaften Kombination des Flexodrucks mit der inline-Messung können Verarbeitungsgeschwindigkeiten von bis zu 100 m/min realisiert werden.

Im Folgenden wird die Erfindung mittels eines Beispiels näher erläutert, ohne jegliche Einschränkung der Erfindung beabsichtigen zu wollen.

### Beispiel

Eine Flexodruckmaschine wurde mit einer verchromten Rasterwalze ausgerüstet, deren Rasterweite 54 Linien pro cm, deren Näpchentiefe 40 µm und deren theoretisches Schöpfvolumen 11,7 cm³/m² beträgt. Als Druckzylinder wurde eine mit EPDM beschichtete Gummiwalze eingesetzt. Ein Gegendruckzylinder bildet mit dem Druckzylinder einen Walzenspalt, in dem der Druck nach der dem Fachmann bekannten Art des Flexodrucks erfolgt. Die Basisrezepturbahn, die über den Gegendruckzylinder in den Walzenspalt geführt wurde, war eine ODF-Basisfilmmaterialrolle in 20 mm Breite und 100 m Länge, deren Hauptbestandteil Hydroxypropylmethylcellulose ist.
Die Druckfarbenlösung bestehend aus 4,4 Gew.-% Hydroxypropylcellulose, 8,9 Gew.-% Arzneiwirkstoff, 2,3 Gew.-% blauem Farbstoff und 84,4 Gew.-% Ethanol wird mit einer Druckgeschwindigkeit von 15 m/min auf die Basisrezepturbahn bedruckt.
Nach dem Druckwerk folgt eine Trocknereinheit, bei der über Konvektionstrocknung das Lösemittel in Form von Ethanol verdampft. Anschließend wird das bedruckte Band zu einer Rolle aufgewickelt und wird - ohne das Flexodruckwerk umzurüsten dreimal hintereinander bedruckt.

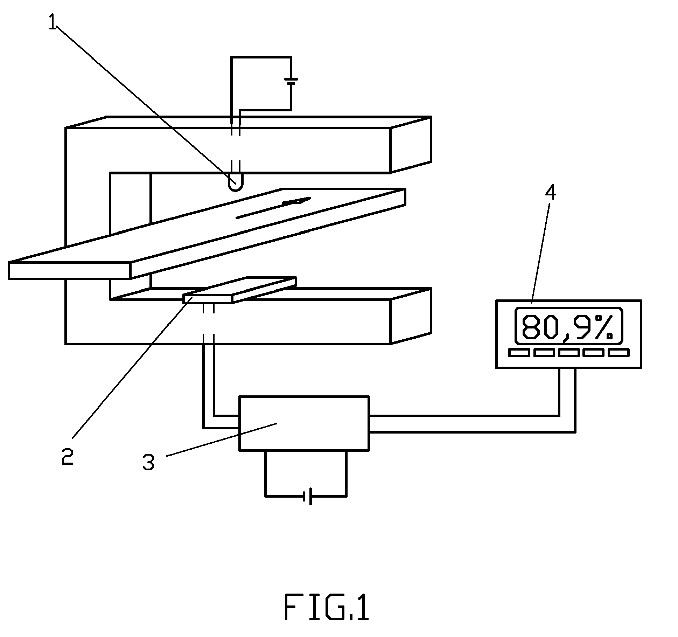

Die ermittelten Transmissionsmesswerte der mit der einen Wirkstoff enthaltenen Farblösung oberseitig bedruckten Basisrezepturbahn wurden mit einer roten Leuchtdiode (= Wellenlänge 650 nm) - wie in Figur 1 dargestellt - gemessen und betrugen:
Ohne Bedruckung = Nullwert = 80,9 %
1x bedruckt: 74,7 %
2x bedruckt: 64,7 %
3x bedruckt: 56,7 %

Die durch ein chemisches Analyseverfahren ermittelte Menge an Arzneiwirkstoff auf der Basisrezepturbahn betrug:
Ohne Bedruckung = Nullwert = 0,00 mg / 6 cm²
1x bedruckt: 0,34 mg / 6 cm²
2x bedruckt: 0,63 mg / 6 cm²
3x bedruckt: 0,87 mg / 6 cm²

Wie zu erkennen ist, nimmt der Transmissionswert mit Zunahme der Farbstoff- und damit einhergehend der Wirkstoffkonzentration ab.
Für eine vorgegebene Konzentration wird der Transmissionswert bestimmt. Abweichungen von diesem außerhalb der vorgegebenen Fehlertoleranzen werden inline und somit sofort festgestellt, so dass ein Eingriff in das Verfahren schnellstmöglich erfolgen kann.

### Bezugszeichenliste

- 1: Rote LED-Lichtquelle
- 2: Photozelle (Empfänger)
- 3: OP-Verstärker
- 4: Anzeige des Transmissionswertes

## Patentansprüche

1. Verfahren zur Herstellung und Kontrolle von oralen Wirkstoff-Filmen mit einer Basis, auf die eine zumindest einen Wirkstoff enthaltende Lösung aufgebracht wird, umfassend die Schritte:
• Dosieren und Mischen der Basisrezeptur,
• Beschichtung der Basisrezeptur auf eine Unterlage, so dass sich eine Bahn ergibt
• gegebenenfalls Trocknung der auf der Unterlage beschichteten Basisrezepturbahn,
• Aufdrucken einer zumindest einen Wirkstoff enthaltenden Farbstofflösung nach dem Flexodruckverfahren auf der Oberseite der Basisrezepturbahn,
• Trocknung der auf der Unterlage beschichteten Basisrezepturbahn samt aufgedruckter Wirkstofflösung,
• Durchstrahlen der auf der Unterlage beschichteten Basisrezepturbahn samt aufgedruckter Wirkstofflösung von der Ober- und/oder Unterseite mittels einer Strahlung aus einer Strahlungsquelle,
• Messung der Transmission der durchgetretenen Strahlung auf der gegenüberliegenden Seite der auf der Unterlage beschichteten Basisrezepturbahn samt aufgedruckter Wirkstofflösung mittels mindestens einer Empfangseinheit.

2. Verfahren nach Anspruch 1,
**dadurch gekennzeichnet, dass**
nach der Beschichtung der Basisrezeptur auf eine Unterlage und der gegebenenfalls anschließenden Trocknung aus der sich ergebenden Bahn die einzelnen Abschnitte der oralen Wirkstoff-Filme gestanzt werden, und zwar entweder direkt vor dem Druckvorgang oder direkt nach dem Druckvorgang.

3. Verfahren nach Anspruch 1,
**dadurch gekennzeichnet, dass**
nach Messung der Transmission der durchgetretenen Strahlung aus der Basisrezepturbahn die einzelnen Abschnitte der oralen Wirkstoff-Filme gestanzt werden.

4. Verfahren nach mindestens einem der Ansprüche 1 bis 3,
**dadurch gekennzeichnet, dass**
die einzelnen Abschnitte der oralen Wirkstoff-Filme abschließend in einen Beutel eingesiegelt werden.

5. Verfahren nach mindestens einem der vorherigen Ansprüche,
**dadurch gekennzeichnet, dass**
als Strahlung Infrarotstrahlung, sichtbares Licht, Ultraviolettstrahlung und/oder radioaktive Strahlung verwendet wird.

6. Verfahren nach mindestens einem der vorherigen Ansprüche,
**dadurch gekennzeichnet, dass**
als Strahlung sichtbares Licht und als Empfangseinheit eine Photozelle verwendet werden.

7. Verfahren nach mindestens einem der vorherigen Ansprüche,
**dadurch gekennzeichnet, dass**
die Basisrezepturbahn auf eine Trennfolie beschichtet wird.

8. Verfahren nach mindestens einem der vorherigen Ansprüche,
**dadurch gekennzeichnet, dass**
die Dicke der Basisrezepturbahn zwischen 25 bis 200 µm, insbesondere zwischen 65 bis 100 µm liegt.

9. Verfahren nach mindestens einem der vorherigen Ansprüche,
**dadurch gekennzeichnet, dass**
das Verfahren kontinuierlich durchgeführt wird.

## Claims

1. Method for producing and monitoring oral dissolvable films comprising a base whereto a solution comprising at least one active compound is applied, said method comprising the steps of
• dosing and mixing the base formulation,
• coating the base formulation onto a support to form a web,
• optionally drying the support-coated base formulation web,
• printing a dye solution comprising at least one active compound onto the upper side of the base formulation web by the flexographic printing process,
• drying the support-coated base formulation web along with the printed active compound solution,
• passing radiation from a radiation source from the upper and/or lower side through the support-coated base formulation web along with the printed active compound solution,
• measuring the transmission of the transmitted radiation using at least one receiving unit on the opposite side of the support-coated base formulation web along with the printed active compound solution.

2. Method according to Claim 1,
**characterized in that**
the step of coating the base formulation onto a support and the optional step of subsequent drying are followed by the step of diecutting the individual portions of the oral dissolvable films out of the resultant web either directly before the printing step or directly after the printing step.

3. Method according to Claim 1,
**characterized in that**
the step of measuring the transmission of the transmitted radiation is followed by the step of diecutting the individual portions of the oral dissolvable films out of the base formulation web.

4. Method according to one or more of Claims 1 to 3,
**characterized in that**
the individual portions of the oral dissolvable films are finally sealed in a pouch.

5. Method according to at least one preceding claim,
**characterized in that**
the radiation used is infrared radiation, visible light, ultraviolet radiation and/or radioactive radiation.

6. Method according to at least one preceding claim,
**characterized in that**
the radiation used is visible light and the receiving unit used is a photocell.

7. Method according to at least one preceding claim,
**characterized in that**
the base formulation web is coated onto a release foil.

8. Method according to at least one preceding claim,
**characterized in that**
the thickness of the base formulation web is between 25 to 200 µm, in particular between 65 to 100 µm.

9. Method according to at least one preceding claim,
**characterized in that**
the method is carried out continuously.

## Revendications

1. Procédé de production et de contrôle de films orodispersibles avec une base, sur laquelle une solution contenant au moins une substance active est déposée, comprenant les étapes suivantes:
• doser et mélanger la recette de base,
• déposer la recette de base sur un substrat, de façon à former une bande,
• éventuellement sécher la bande de recette de base déposée sur le substrat,
• appliquer une solution de colorant contenant au moins une substance active par un procédé d'impression flexographique sur le côté supérieur de la bande de recette de base,
• sécher la bande de recette de base déposée sur le substrat ainsi que la solution de substance active appliquée,
• irradier la bande de recette de base déposée sur le substrat ainsi que la solution de substance active appliquée par le côté supérieur et/ou inférieur au moyen d'un rayonnement provenant d'une source de rayonnement,
• mesurer la transmission du rayonnement ayant traversé sur le côté opposé de la bande de recette de base déposée sur le substrat ainsi que de la solution de substance active appliquée au moyen d'au moins une unité de réception.

2. Procédé selon la revendication 1, **caractérisé en ce que**, après le dépôt de la recette de base sur un substrat et le séchage qui suit éventuellement, on découpe dans la bande résultante les parties individuelles des films orodispersibles, notamment directement avant l'opération d'impression ou directement après l'opération d'impression.

3. Procédé selon la revendication 1, **caractérisé en ce que** l'on découpe les parties individuelles des films orodispersibles dans la bande de recette de base après la mesure de la transmission du rayonnement ayant traversé.

4. Procédé selon au moins une des revendications 1 à 3, **caractérisé en ce que** l'on enferme les parties individuelles des films orodispersibles dans un sachet scellé.

5. Procédé selon au moins une des revendications précédentes, **caractérisé en ce que** l'on utilise comme rayonnement un rayonnement infrarouge, une lumière visible, un rayonnement ultraviolet et/ou un rayonnement radioactif.

6. Procédé selon au moins une des revendications précédentes, **caractérisé en ce que** l'on utilise comme rayonnement une lumière visible et comme unité de réception une cellule photoélectrique.

7. Procédé selon au moins une des revendications précédentes, **caractérisé en ce que** la bande de recette de base est déposée sur un film de séparation.

8. Procédé selon au moins une des revendications précédentes, **caractérisé en ce que** l'épaisseur de la bande de recette de base se situe entre 25 et 200 µm, en particulier entre 65 et 100 µm.

9. Procédé selon au moins une des revendications précédentes, **caractérisé en ce que** le procédé est exécuté en continu.
